# EUROPEAN PATENT APPLICATION

(11) **EP 0 690 077 A1**
(43) Date of publication of application: **03.01.1996**
(21) Application number: 95110169.0
(22) Date of filing: 29.06.1995
(51) Int. Cl.: C08F 20/04, C08L 33/02, C08L 33/14, A61L 15/00, C08F 20/28

(54) **Hydrogel-forming polymeric material**

(30) Priority: 30.06.1994 US 269228
(71) Applicant: KIMBERLY-CLARK CORPORATION, Neenah, Wisconsin 54956-0349 (US)
(72) Inventor: Reeves, William Grover, Appleton, WI 54911 (US)
(74) Representative: Diehl, Hermann, Dr. Dipl.-Phys.

(57) **Abstract**

Disclosed is a hydrogel-forming polymeric material prepared from a first compound selected from the group consisting of ethylenically unsaturated carboxylic acid compounds; and a second compound selected from the group consisting of compounds that comprise a functional group selected from the group consisting of ether and hydroxyl groups. The hydrogel-forming polymeric material exhibits desired absorbent properties and exhibits an improved fracture resistance as compared to the fracture resistance exhibited by an otherwise substantially identical hydrogel-forming polymeric material prepared without the second compound. Also disclosed is a disposable absorbent product containing such a hydrogel-forming polymeric material.

## Description

The present invention relates to a hydrogel-forming polymeric material and an absorbent product incorporating such a material.

The use of hydrogel-forming polymeric materials, commonly known as superabsorbents, in disposable absorbent personal care products is known. Such absorbent materials are generally employed in absorbent products, such as diapers, training pants, adult incontinence products, and feminine care products, in order to increase the absorbent capacity of such products while reducing their overall bulk. Such absorbent materials are generally present in absorbent products in a fibrous matrix, such as a matrix of wood pulp fluff. A matrix of wood pulp fluff generally has an absorbent capacity of about 6 grams of liquid per gram of fluff. The absorbent materials described above generally have an absorbent capacity of at least about 10, preferably of about 20, and often of up to 100 times their weight in physiological saline or human body fluids, such as urine. Clearly, incorporation of such absorbent materials in personal care products can reduce the overall bulk while increasing the absorbent capacity of such products.

A wide variety of materials have been described for use as hydrogel-forming polymeric materials in personal care products. Such hydrogel-forming polymeric materials include synthetic materials, such as polyacrylates. Superabsorbent materials are typically available from various commercial vendors, such as The Dow Chemical Company, Hoechst Celanese, Allied Colloids Limited, or Stockhausen, Inc.

The preparation of hydrogel-forming polymeric materials, such as with a grinding process or by reverse phase polymerization, typically results in a particulate product having a particle-size distribution. Further processing, handling, or treatment of the hydrogel-forming polymeric materials product often subjects the product to additional impact forces. Such impact forces typically result in fracturing of the hydrogel-forming polymeric material particles which results in an increase in small particles. However, in many absorbent products, hydrogel-forming polymeric material particles below a certain size range, such as about 1x10⁻⁴ meters, are generally not acceptable.

During handling and incorporation of the hydrogel-forming polymeric material into disposable absorbent products, the presence of relatively smaller particles often results in airborne particles which can create housekeeping or cleanliness problems, a reduced yield of useable hydrogel-forming polymeric material, and a variance from designed product performance due to variations in anticipated particle size distribution.

The present invention intends to overcome these problems. The object is solved by the hydrogel-forming polymeric material of independent claims 1, 3 and 12 and the disposable absorbent product according to independent claim 18.

Further advantages, features, aspects and details of the invention are evident from the dependent claims, the description and the accompanying drawings. The claims are intended to understood as a first non-limiting approach of defining the invention in general terms. The present invention relates to a hydrogel-forming polymeric material with desirable fracture properties suitable for use in absorbent products. Also, the present invention relates to a disposable absorbent product including an absorbent structure comprising the hydrogel-forming polymeric material.

It is desirable to produce a hydrogel-forming polymeric material, with high absorbent properties but with an increased toughness, such that the hydrogel-forming polymeric material resists fracturing and, thus, substantially retains its particle size distribution during handling and processing.

It is also desirable to prepare a hydrogel-forming polymeric material which would spontaneously form a certain desired particle size distribution during a grinding operation and which would resist further change to the particle size distribution during shipping and handling.

These and other related goals are achieved by a hydrogel-forming polymeric material prepared from a first compound selected from the group consisting of ethylenically unsaturated carboxylic acid compounds and their salts; and a second compound comprising a functional group selected from the group consisting of ether and hydroxyl groups; wherein the hydrogel-forming polymeric material exhibits desired absorbent properties and exhibits an improved fracture resistance as compared to the fracture resistance exhibited by an otherwise substantially identical hydrogel-forming polymeric material that is prepared without the second compound.

In one embodiment of the present invention, a hydrogel-forming polymeric material with desirable fracture properties is prepared from a first compound selected from the group consisting of ethylenically unsaturated carboxylic acid compounds and their salts; and a second compound selected from the group consisting of compounds that comprise a functional group selected from the group consisting of ether and hydroxyl groups; wherein the hydrogel-forming polymeric material exhibits an Absorbency Under Load value of at least about 14 and exhibits a Relative Fracture Resistance value that is at least about 25 percent greater than the Relative Fracture Resistance value exhibited by an otherwise identical hydrogel-forming polymeric material that is prepared without the second compound.

In another aspect, the present invention concerns a disposable absorbent product for the absorption of liquids, such as body liquids.

One embodiment of such a disposable absorbent product comprises a liquid-permeable topsheet, a backsheet attached to the topsheet, and an absorbent structure positioned between the topsheet and the backsheet, wherein the absorbent structure comprises the hydrogel-forming polymeric material with desirable fracture properties described herein.

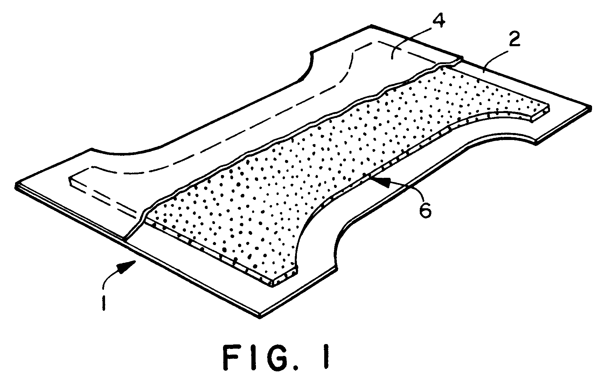

Figure 1 is a perspective view of one embodiment of a disposable absorbent product according to the present invention.

One aspect of the present invention concerns a hydrogel-forming polymeric material prepared from a first compound selected from the group consisting of ethylenically unsaturated carboxylic acid compounds and their salts; and a second compound selected from the group consisting of compounds that comprise a functional group selected from the group consisting of ether and hydroxyl groups.

As used herein, "hydrogel-forming polymeric material" is meant to refer to a high-absorbency material commonly referred to as a superabsorbent material. Such high-absorbency materials are generally capable of absorbing an amount of a liquid, such as synthetic urine, a 0.9 weight percent aqueous saline solution, or bodily fluids, such as menses, urine, or blood, at an amount of at least about 10, suitably about 20, more suitably about 50, and up to about 100 times the weight of the superabsorbent material at the conditions under which the superabsorbent material is being used. Typical conditions include, for example, a temperature of between about 0°C to about 100°C and suitably ambient conditions, such as about 23°C and about 30 to about 60 percent relative humidity. Upon absorption of the liquid, the hydrogel-forming polymeric material typically swells and forms a hydrogel.

The hydrogel-forming polymers are preferably lightly crosslinked to render the material substantially water insoluble yet water swellable. Crosslinking may, for example, be by irradiation or covalent, ionic, van der Waals, or hydrogen bonding.

The first compound useful in preparing the hydrogel-forming polymeric material of the present invention is selected from the group consisting of ethylenically unsaturated carboxylic acid compounds and their respective salts, such as sodium, potassium or ammonium. These compounds may be used either singly or as a mixture of two or more.

As used herein, the term "ethylenically unsaturated carboxylic acid compounds" is intended to represent compounds that comprise, or are prepared from compounds that comprise, both an ethylenically unsaturated and a carboxylic acid functionality. As such, ethylenically unsaturated carboxylic acid compounds are intended to include both monomers that comprise both an ethylenically unsaturated and a carboxylic acid functionality, as well as polymers prepared from such monomers.

Examples of ethylenically unsaturated carboxylic acid compounds useful as the first compound in the present invention include monomers of acrylic acid, methacrylic acid, crotonic acid, maleic acid and its anhydride, fumaric acid, itaconic acid, and the like, their salts, and polymers prepared from these monomers, either singly or as a copolymer of two or more monomers, and their salts.

Suitable for use as the ethylenically unsaturated carboxylic acid compound are monomers of acrylic acid and methacrylic acid, their salts, and polymers prepared from these monomers, either singly or as a copolymer, and their salts.

The second compound useful in preparing the hydrogel-forming polymeric material of the present invention is selected from the group consisting of compounds that comprise a functional group selected from the group consisting of ether and hydroxyl groups. These compounds may be used either singly or as a mixture of two or more compounds. Examples of materials useful as the second compound include poly(ethylene glycol) acrylate, poly(ethylene glycol) methacrylate, poly(propylene glycol) acrylate, poly(propylene glycol) methacrylate, methoxy poly(propylene glycol) acrylate, methoxy poly(propylene glycol) methacrylate, hydroxyethyl acrylate, hydroxyethyl methacrylate, glyceryl acrylate, glyceryl methacrylate, polymethylene oxide, polyethylene oxide, polypropylene oxide, polybutylene oxide, polyvinyl alcohol, polyvinyl pyrollodine, or polymers or copolymers of these compounds.

The second compound is used in an amount effective to provide the hydrogel-forming polymeric material with the desired absorbent and fracture resistant properties described herein. Without wishing to limit the scope of the present invention, it is believed that the fracture resistance of polymers of the first compound are being affected by plasticization of the relatively glassy or brittle first compound polymers by the polyether or polyhydroxyl chains of the comonomer or blended copolymer of the second compound. It is believed that the plasticization results from the polyether or polyhydroxyl chains of the second compound interrupting hydrogen bonding between the unneutralized carboxylic acid functional groups of the first compound. As such, it is believed that any comonomer or copolymer containing available ether or hydroxyl groups capable of interrupting hydrogen bonding of the first compound will be useful as the second compound herein.

The second compound is used in an amount that is beneficially from greater than 0 to about 25 mole percent, suitably from about 0.1 to about 20 mole percent, and more suitably from about 1 to about 15 mole percent per mole of the first compound used. Alternatively, the second compound is used in an amount that is beneficially from greater than 0 to about 200 weight percent, suitably from about 0.1 to about 150 weight percent, and more suitably from about 1 to about 100 weight percent, based on the weight of the first compound used in preparing the hydrogel-forming polymeric material. Alternatively, the second compound is used in an amount that is beneficially from greater than 0 to about 75 weight percent, suitably from about 0.1 to about 50 weight percent, and more suitably from about 1 to about 40 weight percent, based on the total weight of the first and second compounds used in preparing the hydrogel-forming polymeric material.

The first and second compounds may generally be combined by a variety of methods, such as by polymerization, co-polymerization, blending, or a combination of these processes, in order to prepare the hydrogel-forming polymeric material of the present invention.

When polymerization or co-polymerization is used to prepare the hydrogel-forming polymeric material of the present invention, the first or second compounds will generally be used, respectively, as a first or a second monomer. For example, it is possible to prepare a mixture of a first compound monomer and a second compound monomer and then react and co-polymerize the mixture to produce a copolymer comprising both the first and second compound moieties. Alternatively, it is possible to prepare a mixture of a polymer of one of the compounds and a monomer of the other compound and then co-polymerize the mixture to produce a copolymer. The polymerization or co-polymerization of the compounds may generally be carried out by any desired process such as, for example, by bulk, solution, gel, emulsion, suspension, or precipitation polymerization.

Beneficially, a crosslinking agent may be used with the first or second compounds. Examples of suitable crosslinking agents are N,N'-methylbisacrylamide, triallyamine, polyethylene glycol diacrylates, trimethylolpropanetriacrylate, pentaerythritol tri- or tetra-acrylate, and the like. Beneficially, a crosslinking agent is used in an amount from about 0.001 to about 3 mole percent per mole of total first and second monomers or compounds used.

When blending is used to prepare the hydrogel-forming polymeric material of the present invention, the first and second compounds will generally be used, respectively, as a first and a second polymer. The blending of the compounds may generally be carried out by any blending process known to those skilled in the art such as, for example, by hand stirring, mechanical mixing, or the like.

In one embodiment of a blending process useful in preparing the hydrogel-forming polymeric material of the present invention, a first compound polymer is swollen with a solvent, such as water. The swollen first compound polymer is then allowed to solvent exchange with a second compound polymer wherein the second compound polymer is absorbed by the first compound polymer and displaces an amount of the solvent.

One advantage of preparing the hydrogel-forming polymeric material of the present invention by a blending process, as compared to a polymerization process, is that blending processes are generally simpler and more economical than polymerization processes, since appropriate polymers useful in a blend are generally less expensive than the corresponding monomers needed to prepare a copolymer. One disadvantage of preparing a polymer blend, as compared to a copolymer of monomers of the first and second compounds, is that the polymers in a polymer blend can diffuse out upon swelling of the hydrogel-forming polymeric material, thus, potentially reducing the absorbency capacity of the hydrogel-forming polymeric material due to the diffusing polymer increasing the osmotic pressure and the viscosity of the liquid medium being absorbed by the hydrogel-forming polymeric material.

A process combining polymerization and blending may also be used to prepare the hydrogel-forming polymeric material of the present invention. In such a process, for example, a monomer of the first compound may be blended with a polymer of the second compound and then the monomer of the first compound polymerized in situ, forming a blend of polymers of the first and second compounds. Alternatively, a monomer of the second compound may be blended with a polymer of the first compound and then the monomer of the second compound polymerized in situ, forming a blend of polymers of the first and second compounds.

The hydrogel-forming polymeric material, employed in the absorbent products of the present invention, suitably should be able to absorb a liquid under an applied load. For the purposes of this application, the ability of a hydrogel-forming polymeric material to absorb a liquid under an applied load, and thereby perform work, is quantified as the Absorbency Under Load (AUL) value. The AUL value is expressed as the amount (in grams) of an aqueous 0.9 weight percent sodium chloride solution, which the hydrogel-forming polymeric material can absorb in about 60 minutes per gram of hydrogel-forming polymeric material under a load of about 2.0 kPa (about 0.3 pound per square inch), while restrained from swelling in the plane normal to the applied load. The hydrogel-forming polymeric material employed in the absorbent structures or products of the present invention beneficially exhibit an AUL value of at least about 14, more beneficially of at least about 17, suitably of at least about 20, more suitably of at least about 25, and up to about 50. The method by which the AUL value may be determined is set forth in the test methods section herein and also, for example, in detail in US-A-5,149,335 or US-A-5,247,072, the disclosures of which are hereby incorporated by reference.

It has been discovered that, by combining the first and second compounds described herein in a hydrogel-forming polymeric material, it is possible to prepare a hydrogel-forming polymeric material which exhibits high absorbent properties, yet exhibits an increased toughness, such that the material resists fracturing and, thus, substantially retains its particle size distribution during handling and processing. An increased toughness of the hydrogel-forming polymeric material generally means that a lesser amount of relatively smaller particles is produced during processing, shipping, or handling of the hydrogel-forming polymeric material. Such relatively smaller particles represented, for example, by those particles having a size less than about 5x10⁻⁶ meters, often result in airborne particles which can create housekeeping or cleanliness problems, a reduced yield of useable hydrogel-forming polymeric material, and a variance from designed product performance due to variations in anticipated particle size distribution.

As used herein, the "fracture resistance" of a hydrogel-forming polymeric material is meant to represent the ability of the absorbent material to resist fracturing when subjected to an impact. For the purposes of this application, the ability of a hydrogel-forming polymeric material to resist fracturing is quantified as the Relative Fracture Resistance value. The Relative Fracture Resistance value of an evaluated sample is calculated by dividing the amount of particles of a control sample, otherwise substantially identical to the evaluated sample but prepared without the second compound, having a size of 5x10⁻⁶ meters or less, by the amount of particles of the evaluated sample having a size of 5x10⁻⁶ meters or less.

As used herein, the term "otherwise substantially identical hydrogel-forming polymeric material that is prepared without the second compound," and other similar terms, are intended to refer to a control hydrogel-forming polymeric material that is prepared using substantially identical materials and a substantially identical process as compared to a hydrogel-forming polymeric material of the present invention, except that the control hydrogel-forming polymeric material does not comprise or is not prepared with the second compound described herein. As such, the control hydrogel-forming polymeric material generally will not exhibit the desired fracture resistant properties described herein as compared to a hydrogel-forming polymeric material of the present invention.

The hydrogel-forming polymeric material of the present invention exhibits a Relative Fracture Resistance value that is beneficially at least about 25 percent, more beneficially at least about 50 percent, suitably at least about 100 percent, and more suitably at least about 1000 percent greater than the Relative Fracture Resistance value exhibited by an otherwise identical hydrogel-forming polymeric material that does not comprise the second compound.

Suitably, the hydrogel-forming polymeric material is in the form of particles which, in the unswollen state, have maximum cross-sectional diameters within the range of from about 5x10⁻⁵ meters to about 1x10⁻³ meters, more suitably within the range of from about 1x10⁻⁴ meters to about 8x10⁻⁴ meters, as determined by sieve analysis according to American Society for Testing and Materials (ASTM) test method D-1921. It is understood that the particles of hydrogel-forming polymeric material falling within the ranges described above may comprise solid particles, porous particles, or may be agglomerated particles comprising many smaller particles agglomerated into particles falling within the described size ranges.

The hydrogel-forming polymeric materials according to the present invention are suited to absorb many liquids, such as water, saline, and synthetic urine, and body liquids, such as urine, menses, and blood, and are suited for use in disposable absorbent products, such as diapers, adult incontinent products, and bed pads; in catamenial devices such as sanitary napkins, and tampons; and in other absorbent products such as wipes, bibs, wound dressings, and surgical capes or drapes. Accordingly, in another aspect, the present invention relates to a disposable absorbent product comprising a hydrogel-forming polymeric material as described herein.

Use of the described hydrogel-forming polymeric material in disposable absorbent products allows for the formation of a disposable absorbent product which is able to rapidly receive a discharged liquid and yet which product is thin. Typically, the hydrogel-forming polymeric material will be incorporated into a disposable absorbent product in the form of an absorbent structure. Such disposable absorbent products generally comprise a liquid-permeable topsheet, a backsheet, and an absorbent structure, such as an absorbent structure comprising the hydrogel-forming polymeric material of the present invention, located between the topsheet and backsheet.

Exemplary disposable absorbent products are generally described in US-A-4,710,187; US-A-4,762,521; US-A-4,770,656; US-A-4,798,603; and U.S. Serial No. 08/096,654, filed July 22, 1993, in the name of Hansen et al., which references are incorporated herein by reference.

The hydrogel-forming polymeric material is present in an absorbent composition, structure, or product of the present invention in an amount effective to result in the absorbent composition, structure, or product being able to absorb a desired amount of liquid. The hydrogel-forming polymeric material is beneficially present in an absorbent structure of the present invention in an amount of from about 1 to about 100 weight percent, suitably from about 5 to about 99 weight percent, and more suitably from about 10 to about 90 weight percent, based on the total weight of the absorbent structure.

Because the hydrogel-forming polymeric material present in the absorbent structures of the present invention can be present in high concentrations, the absorbent structures of the present invention can be relatively thin and lightweight, have a relatively small volume, and still function in a desirable manner.

The absorbent structure of the present invention suitably comprises a fibrous matrix comprising the hydrogel-forming polymeric material wherein the fibrous matrix constrains or entraps the hydrogel-forming polymeric material.

The fibrous matrix may be formed through an air-laying process, a spunbond or meltblown process, a carding process, a wet-laid process, or through essentially any other process or means, known to those skilled in the art, for forming a fibrous matrix.

Methods of incorporating the hydrogel-forming polymeric material into the fibrous matrix are known to those skilled in the art. Suitable methods include incorporating the hydrogel-forming polymeric material into the matrix during formation of the matrix, such as by air laying the fibers of the fibrous matrix and the hydrogel-forming polymeric material at the same time, or wet-laying the fibers of the fibrous matrix and the hydrogel-forming polymeric material at the same time. It is suitable that the hydrogel-forming polymeric material be generally uniformly distributed within the fibrous matrix. However, the hydrogel-forming polymeric material may be nonuniformly distributed as long as the desired absorbent properties of the absorbent structure are still achieved. Alternatively, it is possible to apply the hydrogel-forming polymeric material to the fibrous matrix after formation of the fibrous matrix. Other methods include sandwiching the hydrogel-forming polymeric material between two sheets of material, at least one of which is fibrous and liquid permeable. The hydrogel-forming polymeric material may be generally uniformly located between the two sheets of material or may be located in discrete pockets formed by the two sheets.

The fibrous matrix may be in the form of a single, integrally formed layer or of a composite comprising multiple layers. If the fibrous matrix comprises multiple layers, the layers are preferably in liquid communication with one another such that a liquid present in one fibrous layer can flow or be transported to the other fibrous layer. For example, the fibrous layers may be separated by cellulosic tissue wrap sheets known to those skilled in the art.

When the fibrous matrix comprises a single, integrally formed layer, the concentration of hydrogel-forming polymeric material may increase along the thickness of the fibrous matrix in a gradual, nonstepwise fashion or in a more stepwise fashion. Similarly, the density may decrease through the thickness in a nonstepwise manner or in a stepwise manner.

The absorbent structures of the present invention may generally be of any size or dimension as long as the absorbent structure exhibits the desired absorbent characteristics.

The absorbent structure of the present invention may also be used or combined with other absorbent structures, with the absorbent structure of the present invention being used as a separate layer or as an individual zone or area within a larger, composite absorbent structure. The absorbent structure of the present invention may be combined with other absorbent structures by methods well known to those skilled in the art, such as by using adhesives or simply by layering the different structures together and holding together the composite structures with, for example, tissue.

In one embodiment of the present invention, a disposable absorbent product is provided, which disposable absorbent product comprises a liquid-permeable topsheet, a backsheet attached to the topsheet, and an absorbent structure comprising a hydrogel-forming polymeric material wherein the absorbent structure is positioned between the topsheet and the backsheet.

While one embodiment of the invention will be described in terms of the use of a hydrogel-forming polymeric material in an infant diaper, it is to be understood that the hydrogel-forming polymeric material is equally suited for use in other disposable absorbent products known to those skilled in the art.

Turning now to the drawing, Fig. 1 illustrates a disposable diaper 1 according to one embodiment of the present invention. Disposable diaper 1 includes a backsheet 2, a topsheet 4, and an absorbent structure 6, located between the backsheet 2 and the topsheet 4. Absorbent structure 6 is an absorbent structure according to the present invention. Specifically, in the illustrated embodiment, absorbent structure 6 comprises a hydrogel-forming polymeric material of the present invention.

Those skilled in the art will recognize materials suitable for use as the topsheet and backsheet. Exemplary of materials suitable for use as the topsheet are liquid-permeable materials, such as spunbonded polypropylene or polyethylene having a basis weight of from about 15 to about 25 grams per square meter. Exemplary of materials suitable for use as the backsheet are liquid-impervious materials, such as polyolefin films, as well as vapor-pervious materials, such as microporous polyolefin films.

Absorbent products and structures according to all aspects of the present invention are generally subjected, during use, to multiple insults of a body liquid. Accordingly, the absorbent products and structures are desirably capable of absorbing multiple insults of body liquids in quantities to which the absorbent products and structures will be exposed during use. The insults are generally separated from one another by a period of time.

### TEST METHODS

### Absorbency Under Load

The Absorbency Under Load (AUL) is a test which measures the ability of an absorbent material to absorb a liquid (0.9 weight percent solution of sodium chloride in distilled water) while under an applied load or restraining force.

To evaluate Absorbency Under Load, a hydrogel-forming polymeric material sample was sieved to obtain a 40 to 50 Tyler-equivalent mesh range particle size. Into a cup, consisting of a rigid plastic tube having a 2.54 cm (1 inch) inner diameter, an outside diameter of 3.2 cm (1.25 inches), and closed at one end with a 100 Tyler-equivalent mesh screen, was placed in a monolayer 0.16 gram of the sieved hydrogel-forming polymeric material sample. The sample is then covered with a plastic spacer disc, weighing about 4.4 grams, which is slightly smaller than the inside diameter of the sample cup and serves to protect the sample from being disturbed during the test. A weight, weighing about 103 grams, was placed onto the spacer disc, thereby applying a load of about 0.02 kg/cm² (about 0.3 pound per square inch) to the hydrogel-forming polymeric material. The total weight of the cup, hydrogel-forming polymeric material, and weight was then determined. The cup was then placed into a dish with sufficient 0.9 weight percent solution of sodium chloride in distilled water to flood the screen and contact the hydrogel-forming polymeric material. The hydrogel-forming polymeric material was allowed to absorb the liquid under the weight load for about 60 minutes. After the 60 minutes, the cup was removed from the dish and the bottom of the cup was blotted on fresh paper toweling, to remove excess fluid, for about 2 minutes. The cup, swollen hydrogel-forming polymeric material, and weight was then reweighed. Any increase in weight was attributed to the hydrogel-forming polymeric material swelling with the saline solution. The weight of saline solution absorbed after 60 minutes is the AUL value expressed as grams saline solution absorbed per gram of absorbent. Generally, three similar hydrogel-forming polymeric material samples are evaluated at the same time and their results averaged to obtain a reportable AUL value.

### Fracture Resistance

The Fracture Resistance is a test which measures the ability of an absorbent material to resist fracturing when subjected to a force due to impact.

To carry out the test, the material to be evaluated is dried at 65°C overnight and then stored in a desiccator over sodium hydroxide pellets until evaluated. The material is then placed in a lab mill (available from the Gilson Company under the trade designation A-10 Lab Mill, made by the Tekmar Company) which is then operated for 120 seconds. Since the lab mill has no controls other than its on/off switch, the operating conditions of the lab mill are essentially preset. The resulting powder is sieved through a stack of 3 inch stainless steel U.S.A. standard testing sieves (A.S.T.M. E-11 specification) with respective Tyler-equivalent mesh sizes of 20, 30, 40, 50, 70, 100, 140, and 200. The weight of powder on each sieve and in the bottom pan is recorded and then converted into the cumulative percentage of the total weight. The cumulative percentages are plotted on a probability scale versus the logarithm₁₀ of the size of the sieve openings. For simple brittle fracture, such a plot is generally a straight line and can be extrapolated to determine the percentage of the material which would fall in a particular size range after fracture, such as the percentage which would be 'fines' or the percentage which could form an aerosol which would require engineering controls in a manufacturing area. A discussion and explanation of particle-size evaluation may be found, for example, in the Instruction Manual for the Series 290 Marple Personal Cascade Impactors, Bulletin No. 290 I.M.-3-82, and the handout "Particle Statistics," D. W. Cooper from the IBM Corporation, presented at the short course "Aerosol Measurement: Science and Technology", at the University of Minnesota, Minneapolis, August 27-30, 1984, such references incorporated herein by reference. In order to evaluate fracture resistance, polymers are compared on the percentage of the distribution which is below 5x10⁻⁶ meters, a size which would require engineering controls if present in a sufficient amount.

Deviations from a straight line indicate preferential fracture along certain planes in the material. Such a deviation is seen in Example 11. Such a preferential fracture plane could be industrially valuable since, as is seen in Example 11, the bulk polymer forms a stable particle size distribution with no fines if particles can be removed from a grinding operation before they are reduced below a size of about 7x10⁻⁵ meters.

To make comparisons between different material samples easier, the amount of particles having a size of 5x10⁻⁶ meters or less, resulting from the fracture resistance evaluation of a control sample prepared without the second compound, may be assigned a Relative Fracture Resistance value of 1.0; and the fracture resistance value of other evaluated samples, otherwise identical to the control sample but prepared with the second compound, can be determined relative to the control sample. The Relative Fracture Resistance value of an evaluated sample is calculated by dividing the amount of particles of the control sample, having a size of 5x10⁻⁶ meters or less, by the amount of particles of the evaluated sample having a size of 5x10⁻⁶ meters or less.

### Examples

### Example 1:

A 75-percent-neutralized sodium polyacrylate was prepared as a control material as follows:
A 1-liter capacity polymer reactor with a 4-hole-top was assembled with a stirrer, nitrogen gas inlet, water-cooled condenser, and a deep-well thermocouple. The apparatus was inserted in a 2-liter heating mantle which was equipped with compressed air cooling connected through the temperature sensor via an electronic temperature controller. The controller was set to maintain the reactor temperature at about 70°C. The apparatus was continuously flushed with nitrogen gas at 100 milliliters/minute during the course of the reaction.

The reactor was charged sequentially with 154 milliliters of water, 15.0 grams (0.375 moles) of sodium hydroxide pellets, 36.0 grams (0.5 moles) of acrylic acid, and 0.77 grams (0.005 mole) of methylenebisacrylamide which was delivered through a powder funnel and washed in with 10 milliliters of water. At this point, the heating mantle was turned on and the time began to be recorded. A solution of 0.2 grams (0.00084 mole) of sodium persulfate (Na₂S₂O₈) in 10 milliliters of water was then prepared and added to the reactor.

Once the reactants had completely gelled, the stirrer and heater were turned off and the time noted. After an additional one hour, the contents of the reactor were emptied and the gel cut into pea-sized dice and dried at 125°C for at least two hours. The dried gel was then bottled for later workup. The yield was approximately 46 grams of product.

The hydrogel-forming polymeric material product was then evaluated for an Absorbency Under Load value and a Relative Fracture Resistance value according to the procedures disclosed herein, and the average results from two testings of the material are shown in Table 1. This control material was assigned a Relative Fracture Resistance value of 1.0.

### Examples 2-8:

Copolymers of 75-percent-neutralized sodium polyacrylate and various ether and hydroxyl containing methacrylate esters were prepared. The process of Example 1 was used except that 10 mole percent, based on the amount of acrylic acid used, of the specific methacrylate ester and an additional 0.08 grams (0.0005 mole) of methylenebisacrylamide, to maintain the molar ratio of total monomer to crosslinker, were also added to the reactor.

The hydrogel-forming polymeric material products were then evaluated for Absorbency Under Load values and Relative Fracture Resistance values according to the procedures disclosed herein and the results are shown in Table 1. Table 1 also shows the monomethacrylate ester comonomer used for each example. For those monomethacrylate ester comonomers containing a polyethylene glycol, the molecular weight of the polyethylene glycol is also shown, in parentheses.

### Example 9:

A copolymer of 75 percent neutralized sodium polyacrylate and poly(propylene glycol) monomethacrylate esters was prepared. The process of Example 1 was used except that 10 grams (22 weight percent, based on the amount of sodium polyacrylate) of poly(propylene glycol) monomethacrylate ester was added to the reactor. In addition, 1.0 gram of a nonionic surfactant, comprising a tetrafunctional block-polymer of polypropylene oxide capped with polyethylene oxide (available from the BASF-Wyandotte Corporation under the trade name Tetronic 1508), was added to the reactor to solubilize the poly(propylene glycol) monomethacrylate.

The hydrogel-forming polymeric material product was then evaluated for Absorbency Under Load values and Relative Fracture Resistance values according to the procedures disclosed herein, and the results are shown in Table 1.

From Table 1, it can be seen that all of the ether or hydroxyl-containing comonomers reduced the amount of aerosol-forming particulates (material with a particle size of about 5x10⁻⁶ meters or less) produced during the grinding of the dry polymeric material. The reduction in the aerosol-forming fraction generally improved with increasing chain length of the polyethylene glycol containing comonomers, and the reduction also improved with increasing hydroxyl content.

From Table 1, it can also be seen that the absorbent property, as represented by the Absorbency Under Load values, of the copolymers is generally reduced as compared to the control sodium polyacrylate. While not wishing to be bound by any particular theory, it is believed that the reduction in absorbency properties may be caused by additional crosslinking due to diacrylate contamination of the monoacrylate starting material. Thus, it may be possible to modify or otherwise optimize the process conditions under which the hydrogen-forming polymeric material of the present invention is prepared so as to improve the absorbent properties while substantially maintaining the desired fracture properties of the hydrogen-forming polymeric material product. For example, a reduction in added crosslinking agent may improve the absorbent properties of the copolymer product. Such an effect is shown in Example 10.

### Example 10:

A copolymer of 75-percent-neutralized sodium polyacrylate and polyethylene glycol methacrylate ester (wherein the polyethylene glycol had a molecular weight of about 400) was prepared. The process of Example 1 was used except that 24.2 grams (0.05 mole) of the polyethylene glycol methacrylate ester and only 0.29 grams (0.0019 mole) of methylenebisacrylamide were added to the reactor.

The hydrogel-forming polymeric material product was then evaluated for Absorbency Under Load values and Relative Fracture Resistance values according to the procedures disclosed herein and the results shown in Table 1.

As can be seen from Table 1, the Absorbency Under Load value of Example 10 improved as compared to Example 6; although the Relative Fracture Resistance value decreased. The only difference between Example 10 and Example 6 was the amount of crosslinking agent used.

### Examples 11-14:

The effect of changing the mole percent of the comonomer of the second compound used was evaluated. The process of Example 1 was used except that varying amounts of methoxy polyethylene glycol methacrylate ester (wherein the methoxy polyethylene glycol had a molecular weight of about 1000) were added to the reactor. The hydrogel-forming polymeric material products were then evaluated for Absorbency Under Load values and Relative Fracture Resistance values according to the procedures disclosed herein, and the results are shown in Table 1.

From Table 1, it can be seen that there is relatively little difference in the fracture properties of either Example 12 or 13 (respectively with 5 and 10 mole percent methoxy polyethylene glycol methacrylate ester), while there were dramatic differences in dust produced during the fracturing of examples 11 and 14 (respectively with 1 and 15 mole percent methoxy polyethylene glycol methacrylate ester). Example 14 was rubbery at room temperature and may have been cut, rather than fractured, by the action of the lab mill.

Example 11 is particularly interesting. In the analysis, the plot of cumulative weight percent versus the logarithm of the size of the sieve showed two separate lines which intersected at about 7x10⁻⁵ meters. Large pieces of the material cleanly fractured into particles down to about 70 mesh size, but particles below this 70 mesh size were apparently extremely brittle and shattered, rather than fractured, giving essentially 100 percent conversion into particles smaller than 5x10⁻⁶ meters. Such a result suggests that, if particles could be removed while still above the 70 mesh size, processing of the material could be essentially dust free.

### Examples 15-16:

The process of Example 1 was used, except that varying amounts of polyethylene glycol methacrylate ester (wherein the polyethylene glycol had a molecular weight of about 400) and no methylenebisacrylamide were added to the reactor.

The hydrogel-forming polymeric material products were then evaluated for Absorbency Under Load values and Relative Fracture Resistance values according to the procedures disclosed herein and the results are shown in Table 1.

### Examples 17-18:

The effect of using a polymer of the second compound as a blending agent with a polyacrylate polymer is evaluated. The process of Example 1 was used except that varying weights of polyethylene glycol (wherein the polyethylene glycol had a molecular weight of about 8000; available from Union Carbide Corporation) were added to the reactor in addition to the acrylic acid. The hydrogel-forming polymeric material products were then evaluated for Absorbency Under Load values and Relative Fracture Resistance values, according to the procedures disclosed herein, and the results shown in Table 1.

From Examples 17 and 18, it can be seen that an ether-containing polymer, added before the polymerization of the sodium polyacrylate, will result in an increased resistance to particle fracture.

### Example 19:

The process of Example 1 was used, except that the prepared sodium polyacrylate was diced after its preparation. The diced material was then added to an excess of polyethylene glycol (wherein the polyethylene glycol had a molecular weight of about 600). After the diced material had dehydrated in the polyethylene glycol, the solid was separated by filtration, washed repeatedly in acetone to remove any polyethylene glycol residues external to the sodium polyacrylate material, and allowed to air dry. The hydrogel-forming polymeric material product was then evaluated for Absorbency Under Load values and Relative Fracture Resistance values, according to the procedures disclosed herein, and the results shown in Table 1.

While the invention has been described in detail with respect to specific embodiments thereof, it will be appreciated that those skilled in the art, upon attaining an understanding of the foregoing, may readily conceive of alterations to, variations of and equivalents to these embodiments. Accordingly, the scope of the present invention should be assessed as that of the appended claims and any equivalents thereto.

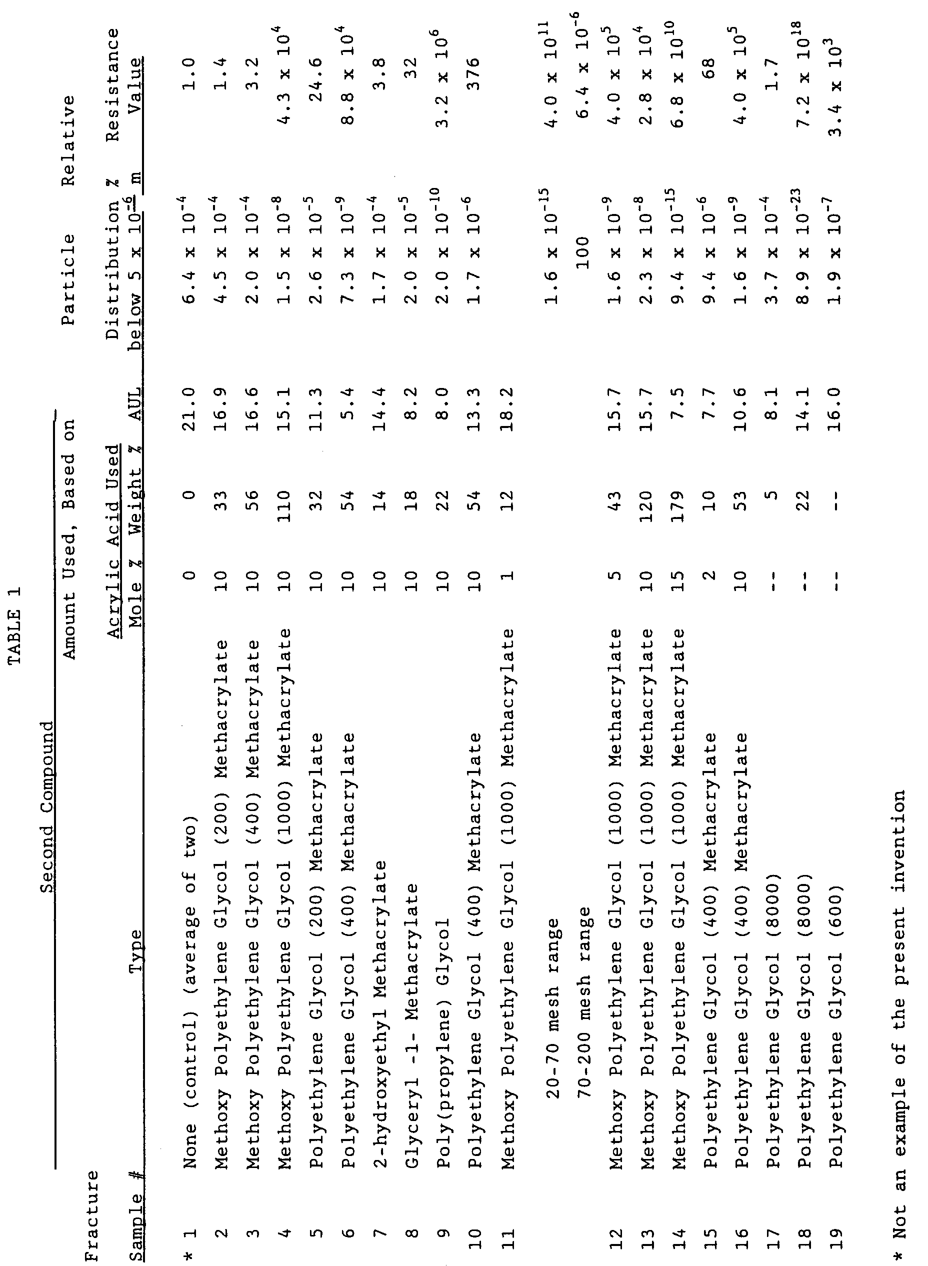

## Claims

1. A hydrogel-forming polymeric material prepared from:
a. a first compound selected from the group consisting of ethylenically unsaturated carboxylic acid compounds and their salts; and
b. a second compound comprising a functional group selected from the group consisting of ether and hydroxyl groups;
wherein the hydrogel-forming polymeric material exhibits an Absorbency Under Load value of at least about 14 and exhibits a Relative Fracture Resistance value that is at least about 25 percent greater than the Relative Fracture Resistance value exhibited by an otherwise substantially identical hydrogel-forming polymeric material that is prepared without the second compound.

2. The hydrogel-forming polymeric material of claim 1 wherein the first compound is selected from the group consisting of acrylic acid, methacrylic acid, crotonic acid, maleic acid and its anhydride, fumaric acid, itaconic acid, their salts, and their polymers.

3. A hydrogel-forming polymeric material especially according to one of claims 1 or 2 wherein the hydrogel-forming polymeric material is a polymer prepared from:
a. a first monomer selected from the group consisting of ethylenically unsaturated carboxylic acid compounds and their salts; and
b. a second compound comprising a functional group selected from the group consisting of ether and hydroxyl groups;
wherein the hydrogel-forming polymeric material exhibits an Absorbency Under Load value of at least about 14 and exhibits a Relative Fracture Resistance value that is at least about 25 percent greater than the Relative Fracture Resistance value exhibited by an otherwise substantially identical hydrogel-forming polymeric material that is prepared without the second compound.

4. The hydrogel-forming polymeric material of claim 3 wherein the first monomer is selected from the group consisting of acrylic acid, methacrylic acid, crotonic acid, maleic acid and its anhydride, fumaric acid, itaconic acid, and their salts.

5. The hydrogel-forming polymeric material of one of the preceding claims wherein the hydrogel-forming polymeric material exhibits an Absorbency Under Load value of at least about 17, preferably of at least about 20.

6. The hydrogel-forming polymeric material of one of the preceding claims exhibiting a Relative Fracture Resistance value that is at least about 50 percent, preferably at least about 100 percent and most preferably at least about 1000 percent greater than the Relative Fracture Resistance value exhibited by an otherwise substantially identical hydrogel-forming polymeric material prepared without the second compound.

7. The hydrogel-forming polymeric material of one of the preceding claims wherein the second compound is selected from the group consisting of poly(ethylene glycol) acrylate, poly(ethylene glycol) methacrylate, poly(propylene glycol) acrylate, poly(propylene glycol) methacrylate, methoxy poly(propylene glycol) acrylate, methoxy poly(propylene glycol) methacrylate, hydroxyethyl acrylate, hydroxyethyl methacrylate, glyceryl acrylate, and glyceryl methacrylate.

8. The hydrogel-forming polymeric material of one of the preceding claims wherein the second compound is used in an amount that is from greater than 0 to about 25 mole percent per mole of the first compound used.

9. The hydrogel-forming polymeric material of one of the preceding claims wherein the second compound is used in an amount that is from greater than 0 to about 200 weight percent, preferably from greater than 0 to about 75 weight percent, based on the weight of the first compound used in preparing the hydrogel-forming polymeric material.

10. The hydrogel-forming polymeric material of one of claims 3 to 9 wherein the second compound is a polymer prepared from the group consisting of poly(ethylene glycol) acrylate, poly(ethylene glycol) methacrylate, poly(propylene glycol) acrylate, poly(propylene glycol) methacrylate, methoxy poly(propylene glycol) acrylate, methoxy poly(propylene glycol) methacrylate, hydroxyethyl acrylate, hydroxyethyl methacrylate, glyceryl acrylate, and glyceryl methacrylate.

11. The hydrogel-forming polymeric material of one of claims 3 to 10 wherein the second compound is used in an amount that is from greater than 0 to about 25 mole percent per mole of the first monomer used.

12. A hydrogel-forming polymeric material especially according to one of the preceding claims, wherein the hydrogel-forming polymeric material is a polymer blend comprising:
a. a first polymer prepared from the group consisting of ethylenically unsaturated carboxylic acid compounds and their salts; and
b. a second polymer comprising a functional group selected from the group consisting of ether and hydroxyl groups;
wherein the hydrogel-forming polymeric material exhibits an Absorbency Under Load value of at least about 14 and exhibits a Relative Fracture Resistance value that is at least about 25 percent greater than the Relative Fracture Resistance value exhibited by an otherwise substantially identical hydrogel-forming polymeric material that does not comprise the second compound.

13. The hydrogel-forming polymeric material of claim 12 wherein the first polymer is prepared from the group consisting of acrylic acid, methacrylic acid, crotonic acid, maleic acid and its anhydride, fumaric acid, itaconic acid, and their salts.

14. The hydrogel-forming polymeric material of claim 12 or 13 wherein the second polymer is selected from the group consisting of polymethylene oxide, polyethylene oxide, polypropylene oxide, polybutylene oxide, polyvinyl alcohol, polyvinyl pyrollodine, or copolymers thereof.

15. The hydrogel-forming polymeric material of claims 12 to 14 wherein the second polymer is used in an amount that is from greater than 0 to about 200 weight percent, based on the weight of the first polymer used.

16. The hydrogel-forming polymeric material of claims 12 to 15 wherein the hydrogel-forming polymeric material exhibits an Absorbency Under Load value of at least about 17.

17. The hydrogel-forming polymeric material of claims 12 to 16 exhibiting a Relative Fracture Resistance value that is at least about 50 percent, preferably at least about 100 percent and most preferably at least about 1000 percent greater than the Relative Fracture Resistance value exhibited by an otherwise substantially identical hydrogel-forming polymeric material prepared without the second polymer.

18. A disposable absorbent product especially according to one of the preceding claims, comprising:
a liquid-permeable topsheet, a backsheet attached to the topsheet, and an absorbent structure positioned between the topsheet and the backsheet, wherein the absorbent structure comprises a hydrogel-forming polymeric material prepared from:
a. a first compound selected from the group consisting of ethylenically unsaturated carboxylic acid compounds and their salts; and
b. a second compound comprising a functional group selected from the group consisting of ether and hydroxyl groups;
wherein the hydrogel-forming polymeric material exhibits an Absorbency Under Load value of at least about 14 and exhibits a Relative Fracture Resistance value that is at least about 25 percent greater than the Relative Fracture Resistance value exhibited by an otherwise substantially identical hydrogel-forming polymeric material that is prepared without the second compound.

19. The disposable absorbent product of claim 18 wherein the first compound is selected from the group consisting of acrylic acid, methacrylic acid, crotonic acid, maleic acid and its anhydride, fumaric acid, itaconic acid, their salts, and their polymers.

20. The disposable absorbent product of claim 18 or 19 wherein the second compound is selected from the group consisting of poly(ethylene glycol) acrylate, poly(ethylene glycol) methacrylate, poly(propylene glycol) acrylate, poly(propylene glycol) methacrylate, methoxy poly(propylene glycol) acrylate, methoxy poly(propylene glycol) methacrylate, hydroxyethyl acrylate, hydroxyethyl methacrylate, glyceryl acrylate, and glyceryl methacrylate, polymethylene oxide, polyethylene oxide, polypropylene oxide, polybutylene oxide, polyvinyl alcohol, polyvinyl pyrollodine, or polymers or copolymers thereof.

21. The disposable absorbent product of claims 18 to 20 wherein the second compound is used in an amount that is from greater than 0 to about 25 mole percent per mole of the first compound used.

22. The disposable absorbent product of claims 18 to 21 wherein the second compound is used in an amount that is from greater than 0 to about 200 weight percent based on the weight of the first compound used.

23. The disposable absorbent product of claims 18 to 22 wherein the hydrogel-forming polymeric material exhibits an Absorbency Under Load value of at least about 17, preferably of at least about 20.

24. The disposable absorbent product of claims 18 to 23 wherein the hydrogel-forming polymeric material exhibits a Relative Fracture Resistance value that is at least about 50 percent, preferably at least about 100 percent and most preferably at least about 1000 percent less than the Relative Fracture Resistance value exhibited by an otherwise substantially identical hydrogel-forming polymeric material prepared without the second compound.
